# EUROPEAN PATENT SPECIFICATION

(11) **EP 1 624 076 B1**
(45) Date of publication and mention of the grant of the patent: **28.02.2007**
(21) Application number: 04292012.4
(22) Date of filing: 06.08.2004
(51) Int. Cl.: C12Q 1/70

(54) **Quantitative detection of HDV by real-time RT-PCR**
RT-PCR-Verfahren zum quantitativen Nachweis von HDV
Procédé de RT-PCR pour la détection quantitative d'HDV

(43) Date of publication of application: 08.02.2006
(73) Proprietor: ASSISTANCE PUBLIQUE - HOPITAUX DE PARIS, 75004 Paris RP (FR)
(72) Inventor: Deny, Paul, 78420 Carrières sur Seine (FR); Gault, Elyanne, 75017 Paris (FR); Le Gal, Frédéric, 77410 Claye Souilly (FR)
(74) Representative: Vialle-Presles, Marie José

(56) References cited:
- WO-A-03/027291
- US-A- 5 225 337
- US-A- 5 625 047
- OMBANDZA-MOUSSA E ET AL: "Hepatitis delta virus RNA detection by one-step RT-PCR" ANNALES DE BIOLOGIE CLINIQUE, vol. 62, no. 3, May 2004 (2004-05), pages 319-324, XP009044662 ISSN: 0003-3898
- WU J-C ET AL: "Characterisation and phylogenetic analysis of a novel hepatitis D virus strain discovered by restriction fragment length polymorphism" JOURNAL OF GENERAL VIROLOGY, SOCIETY FOR GENERAL MICROBIOLOGY, READING, GB, vol. 79, 1998, pages 1105-1113, XP002196403 ISSN: 0022-1317

## Description

The present invention relates to specific reagents for quantitative assay of HDV and more specifically to follow HDV viral load in chronically infected patients.

The present invention also relates to such a quantitative assay.

The hepatitis Delta Virus (HDV) is responsible for severe acute and chronic hepatitis in patients previously or co-infected with hepatitis B virus. The treatment relies on long-term administration of high-doses of alpha-interferon (IFN) and its efficacy is usually followed by the detection of specific anti-HDV IgM or HDV genome in serum.

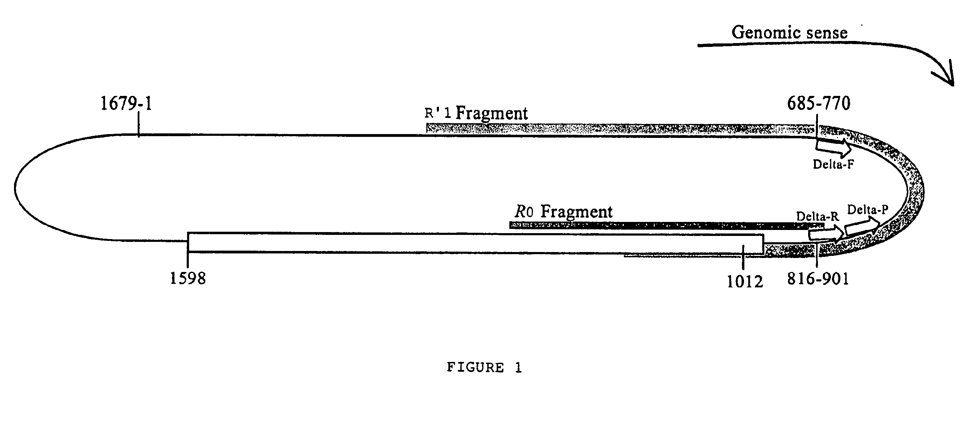

Hepatitis Delta virus (HDV) is a 36 nm viral particle which depends on the hepatitis B virus (HBV) for virion assembly and propagation ¹. HDV particle consists of a circular single-stranded RNA genome of 1,672 to 1,697 nucleotides ^{2,3}, which assembles with two viral proteins, sHD and LHD, to form the ribonucleoprotein. In the course of viral replication, the ribonucleoprotein buds through the hepatocyte endoplasmic reticulum, and acquires an envelop in which the hepatitis B surface antigens (HBsAg) are embedded. Extensive intramolecular complementarity leads to base pairing and gives the HDV genome a pseudo-double strand structure ³ (Figure 1). Extensive sequence analyses of numerous isolates have lead to the classification of HDV in at least 7 distinct clades, with different geographic distributions ². Briefly, HDV clade 1 viruses (genotype I, hereafter named HDV-1) are found in most areas, clades 2 and 4 (genotypes IIA and IIB respectively, referred to as HDV-2 and HDV-4) are located in far eastern areas, clade 3 (genotype III, HDV-3) in the north of South America, and clades 5, 6 and 7 (HDV-5, HDV-6, and HDV-7), in Western Africa ^{2,4-7} (see also WO 03/027291).

HDV infection can cause severe liver disease, with fulminant hepatitis occurring at least 10 times more often than with HBV alone, and a chronicity rate reaching 70 to 90% in case of super-infection ^{1,8,9}. In many cases, chronic delta hepatitis evolves to cirrhosis (60-70%) and liver cancer ^{10,11}, and the treatment efficacy is disappointing ^{12,13}. Indeed, after 12 months interferon-alpha (IFN) at high dose (27 MU weekly) (which is the only regimen which seems to increase long-term survival ¹⁴), only about 50% patients respond to the treatment, and 40% of the responders relapse during the 6 months following the end of therapy ¹². Neither lamivudine, a nucleosidic analog inhibitor of the HBV-DNA polymerase ^{15,16}, nor ribavirin, acyclovir, or famciclovir are efficient in the control of HDV infection ¹⁷⁻¹⁹.

The diagnosis of HDV infection usually relies on the detection of specific anti-HDV antibodies, with the presence of anti-HDV IgM reflecting ongoing viral replication. However, this serological approach for the detection of virus replication lacks sensitivity ^{20,21}.

A sum up of the evolution of the B and delta markers during co-infections and superinfection is presented in the PCT International Application WO 03/027291.

HDV antigen is rarely detected in the serum, except in the case of acute infection (prior to the antibody sero-conversion), or among severely immuno-suppressed chronically infected patients ²². Therefore, HDV replication is most efficiently evaluated by the detection of HDV-RNA in serum, using home-made qualitative RT-PCR assays ²³. Although HDV-RNA detection in serum is usually related to liver damage and poor outcome ²⁴, it does not allow to evaluate treatment efficacy as precisely as with a quantitative approach ²⁵.

Real-time PCR provides an accurate and sensitive means of quantifying viral genomes, with the major advantage of avoiding post-PCR handling that can be source of DNA carryover. Several studies have reported the benefit of this technique for the quantification of viral genomes in blood samples ²⁶⁻²⁸. Recently, Yamashiro T. et al. developed a real-time RT-PCR assay to quantify HDV-RNA in serum, and suggested a possible correlation between viral load and clinical stage of the liver disease ²⁹. The primers used in Yamashiro T. et al. were designed on the basis of a region conserved among type 1, 2a and 2b HDV genotypes. Therefore, said primers do not take in account the other HDV genotypes.

General principles of real time quantitative RT PCR are known in the Art and are for instance described in Poitras et al. ⁴³ and Gibson et al.⁴⁴.

Real-time PCR based on the TaqMan® technology allows DNA or cDNA quantification over a large dynamic range (10 to 10⁷ copies), and is therefore well adapted to the quantification of viral genomes. In the case of HDV, such a large range of quantification is required, considering that very high viral loads have been detected in chimpanzees (during the acute phase of infection after direct liver inoculation) ³⁶, and that treatment follow-up requires the possibility to detect very low amounts of RNA. Moreover, the possibility of handling numerous samples and the absence of post-PCR handling makes it a safe and convenient approach for clinical diagnosis.

However none of the already proposed methods is able to detect with a good sensitivity and a high specificity any HDV genotype including type 3 and the recently described types 5, 6 and 7.

Moreover, the development of an accurate and sensitive test for HDV-RNA quantification in blood samples raises two technical problems. First, the "rod-like" structure of HDV-RNA, which is due to an intramolecular base pairing of over 70% of the sequence ^{3,37}, tends to impair cDNA synthesis efficacy, and therefore, PCR efficacy. Second, the genetic variability of the virus ^{2,4-7} implies a specific design of primers and probe. Indeed, the divergence between HDV types has been shown to be as high as 37% over the entire nucleotide sequence of the genome ².

The Inventors have therefore given themselves the aim of providing HDV nucleic acid molecules reagents capable of being carried out in an assay which is able to quantify HDV-RNA of all HDV types in the serum of infected patients. HDV-RNA quantification, in serum, with reagents of the instant invention selected within the ribozymes, constitutes unexpectedly an improvement in the follow-up of patients over the art.

Indeed, primers and probe designed in these regions, despite the strong secondary structures they contain, lead to a PCR efficacy over 93%.

Furthermore, it will help to understand the natural history of HDV infection and to define guidelines for the management of chronic delta hepatitis.

Said assay was, unexpectedly, sensitive enough to detect 100 copies of HDV-RNA per ml of serum, was reproducible, and was efficient to detect the genome of all HDV types including type 3 and the recently described types 5, 6 and 7. Viral load determination in treated patients would be helpful to discriminate responders from non-responders to IFN therapy, with more accuracy than with either the usual qualitative approach or the quantitative approach based on the use of primers selected in the delta antigen coding region.

The subject-matter of the present invention is therefore a method for detecting HDV-RNA in a biological sample, which method is characterized in that it comprises:
(1) a step of extracting HDV-RNA from a biological sample and
(2) a step of evaluating the quantity of said HDV-RNA by real-time RT-PCR quantification comprising:
   - amplifying its corresponding cDNA in the presence of at least a pair of primers selected from the group consisting of the following pairs:
      (a) Delta-F (direct primer): 5'-GCATGGTCCCAGCCTCC-3'(SEQ ID NO:1) and Delta-R (reverse primer): 5'-TCTTCGGGTCGGCATGG-3' (SEQ ID NO:2) and
      (b) T3-Delta-F (direct primer): 5'-GCATGGCCCCAGCCTCC-3' (SEQ ID NO:3) and Delta-R (reverse primer): 5'-TCTTCGGGTCGGCATGG-3' (SEQ ID NO:2). Because of the existence of one mismatch with the sequences of type III genome, a second direct primer was specifically designed for the amplification of HDV-3 isolates: T3-Delta-F: 5'-GCATGGCCCCAGCCTCC-3' (SEQ ID NO:3); therefore the here above mentioned two pairs of primers may be advantageously used simultaneously; and
   - detecting the quantity of the formed amplicons and comparing said quantity with at least a control sample.

According to an advantageous embodiment of said method, the detecting step is performed in the presence of a fluorogenic probe [Delta-P (probe)] consisting of the following sequence: 5'- ATGCCCAGGTCGGAC-3' (SEQ ID NO:4).

According to another advantageous embodiment of said method, said detecting step includes as control sample, a positive control sample selected in the group consisting of R' 1 region of an HDV genome, corresponding to positions 305-1161, (see International PCT Application WO 03/027291), a fragment of said region R'1 comprising the amplicon (positions 692-904) and the whole genome of an HDV, said positive control being preferably in the form of a plasmid.

According to another advantageous embodiment of said method, said detection step includes as control sample, a positive control sample as defined here above and a negative control (serum sample negative for HDV, for instance).

The positions of the different primers and of the probe (SEQ ID NO:1-4) on the HDV genome are illustrated on Figure 1, the positions in the HDV genome, being indicated on the circular genome in genomic orientation, according to the numbering of Wang et al. ³, of the HDV genome.

The positions of the primers are selected in view to obtain an amplicon having about 215-221 bp. The sequences of the primers are selected in view to obtain a specific hybridization: no cross hybridization, Ta (Temperature of annealing) of about 60°C and high GC content; moreover, said primers have been designed to obtain high efficacy in the conserved secondary structure of the ribozyme.

Advantageously, said fluorogenic probe is labeled at least at one of its ends by a fluorescent dye. Preferably, the 5' end is labeled with a fluorescent reporter dye and the 3' end is labeled with a quencher dye.

Moreover, one of said end, preferably the 3' end of said probe is also labeled with a MGB (Minor Groove Binder) group, which increases artificially the hybridization temperature of the probe and therefore makes possible the use of probes of reduced length.

Useful fluorescent reporter dyes are known in the art; they are chosen, for instance, among the followings: 6-FAM (6-carboxyfluorescein), TET (tetrachloro-6-carboxyfluorescein), HEX (hexachloro-6-carboxy-fluorescein), fluorescein isothyocyanate (FITC), rhodamine, cyanine (CY3, CY5) and Texas red¹².

Useful quencher dyes are also known in the art; they are chosen, for instance among the followings: methyl red or TAMRA (6-carboxytetramethylrhodamine) ⁴² or dark quenchers.

A preferred probe according to the instant invention is the following one: 5'-FAM-ATGCCCAGGTCGGAC-MGB-3' (= 5'-FAM-SEQ ID NO:4-MGB-3').

The instant real-time RT-PCR assay is sensitive enough to detect 100 HDV-RNA copies per ml serum, whatever the HDV type tested, and was used to quantify viral loads in sequential sera collected from patients with chronic delta hepatitis and previously treated with IFN. The results obtained show that treatment efficacy can be monitored with much more accuracy with a quantitative approach combined with the instant specific primers and probe. Thus, the instant method constitutes an improvement for the management of chronically infected patients and should help to define standardized treatment guidelines.

The instant quantitative real-time RT-PCR method is able to replace the qualitative assay in the routine diagnosis procedure. This is made possible because of the sensitivity of the method, and because of its potential ability to detect HDV-RNA of all known types, avoiding false negative results. Concerning HDV-3 sequences, the existence of a mismatch with one of the primers has led us to use a specific forward primer when a HDV-3 infection is suspected (considering the clinical datas, the geographical origin of the patient, and the non-detection of HDV-RNA despite the presence of specific anti-HDV IgM). Both pairs of primers may be used.

The possibility to detect HDV-RNA in serum or plasma is an improvement for patient's follow-up, as compared to IgM detection, which is specific but not sensitive enough ³⁸. When a qualitative RT-PCR is performed, the intensity of the signal obtained with ethidium bromide staining of the amplicons (and eventually the following Southern blot) can give a rough indication on the viral load, but it remains imprecise and will not allow discriminating between viral loads exceeding 50,000 copies/ml plasma.

A subject-matter of the present invention is also a detection kit, characterized in that it comprises further to the buffers and the reagents necessary for extracting HDV-RNA from a biological sample, synthetizing its corresponding cDNA and amplifying said cDNA, at least two pairs of primers as defined here above.

According to an advantageous embodiment of said kit, it further comprises:
- a fluorogenic probe, as defined here above,
- a positive control sample selected in the group consisting of R' 1 region of an HDV genome corresponding to positions 305-1161, a fragment of said R' 1 region comprising the amplicon (positions 692-904) and the whole genome of an HDV and
- a negative control, such as a serum negative for HDV.

According to an advantageous embodiment of said kit, said fluorogenic probe is labeled at its 5' end with a fluorescent reporter dye and at its 3' end with a quencher dry, preferably a dark quencher and a MGB group.

Another subject-matter of the present invention is also pairs of primers able to amplify any HDV genome, said pairs of primers being selected in the group consisting of the pair of primers SEQ ID NO:1 and SEQ ID NO:2 and the pair of primers SEQ ID NO:3 and SEQ ID NO:2.

Another subject-matter of the present invention is also a probe specific of the nucleic acid fragment amplified by the here above mentioned pairs of primers, said probe consisting of the sequence defined by SEQ ID NO:4.

Besides of the above arrangements, the invention also comprises other arrangements which will emerge from the following description, which refers to examples of implementation of the present invention and also to the attached drawings in which:
- Figure 1 is a schematic representation of the HDV genome. HDV genome is a circular single-stranded RNA molecule of approximately 1680 nucleotides. It is classically represented as a pseudo-double stranded rod-like molecule, due to an extensive intramolecular base pairing. The delta antigen coding region (on the antigenomic strand) is represented as a white rectangle, with the edition site at the position 1012 (according to Wang et al ³). Ribozymes are located at nucleotide 900/901 (anti-genome ribozyme) and 685/686 (genome ribozyme). The *R0* region (900-1280), amplified with the qualitative RT-PCR assay, and *R1* (305-1161), which was cloned in pCRII and used as a standard for quantification in the real-time RT-PCR assay, are represented as grey lines. Forward (Delta-F) and reverse (Delta-R) primers and probe (Delta-P) are represented as empty arrows.

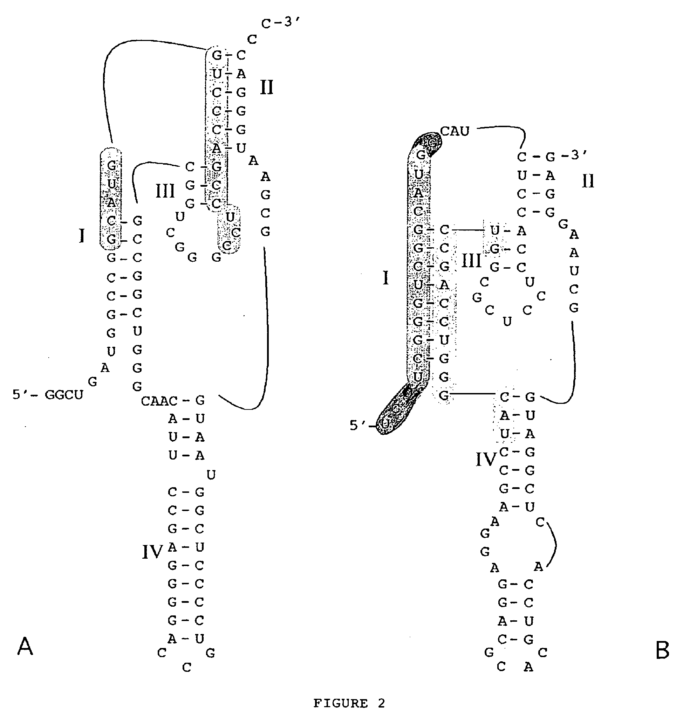

- Figure 2 represents primers and probe designed in the ribozyme regions of the HDV genome (according to Perrotta et al ⁴¹)
   A: Genome ribozyme: the forward primer sequence is highlighted in dark grey.
   B: Anti-genome ribozyme: the reverse primer sequence is highlighted in dark grey, the probe in light grey.

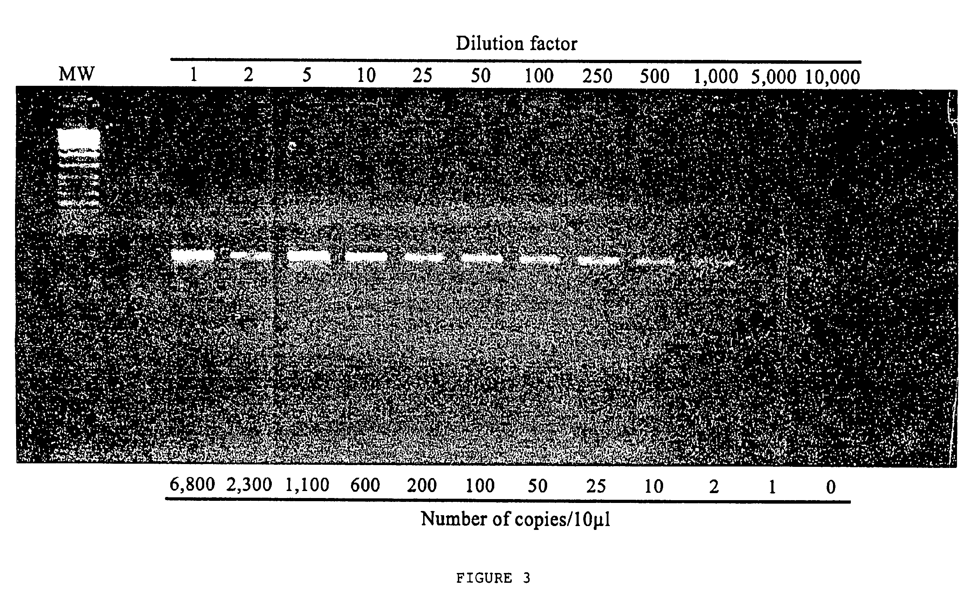

- Figure 3 represents comparative sensitivities of the qualitative and quantitative RT-PCR assays. Example of sample n°40. End-point dilutions (10 µl) of cDNA (sample n°40) were tested both with the qualitative and the quantitative assay. With the qualitative assay, the last visible dilution after ethidium bromide staining of a 1.5% agarose gel was 1/1,000. With the quantitative real-time PCR assay, 1 copy was detected in 10 µl of cDNA diluted at 1/5,000.

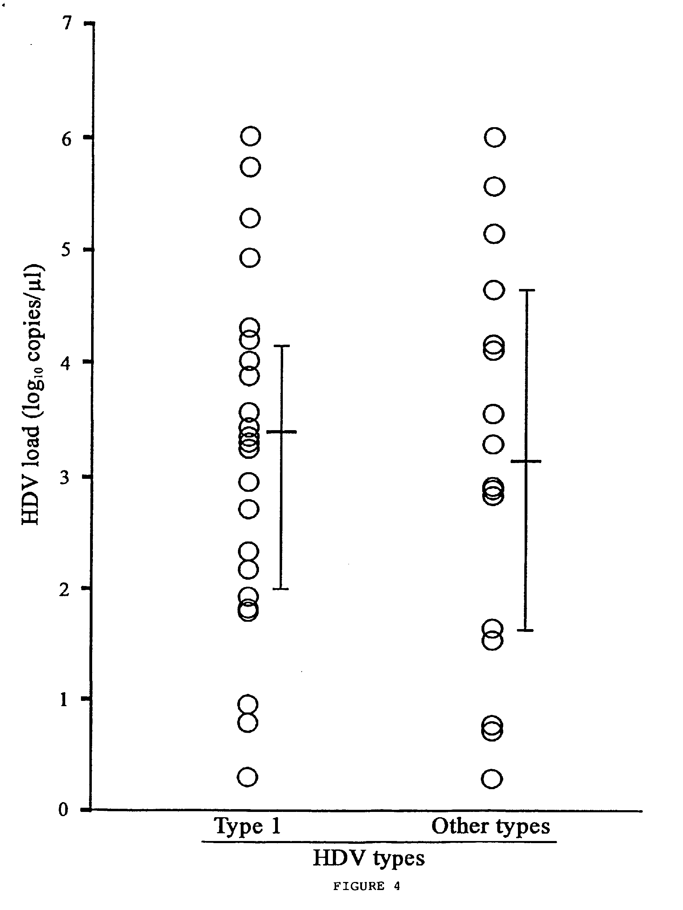

- Figure 4 illustrates a range of viral loads obtained from a panel of various HDV clades; twenty three HDV-1 samples were tested. The median viral load was 1,950 copies/10 µl cDNA (ranging from 2 to 986,000 copies/10 µl cDNA). Sixteen HDV non-1 were tested (HDV-2, n=1, HDV-5, n=6, HDV-6, n=4, HDV-7, n=5). The median viral load was 1,300 copies/10µl cDNA (ranging from 2 to 1,000,000 copies/10 µl cDNA). No significant difference was detected between HDV-1 and HDV-nonl load (Mann Whitney non parametric U test).

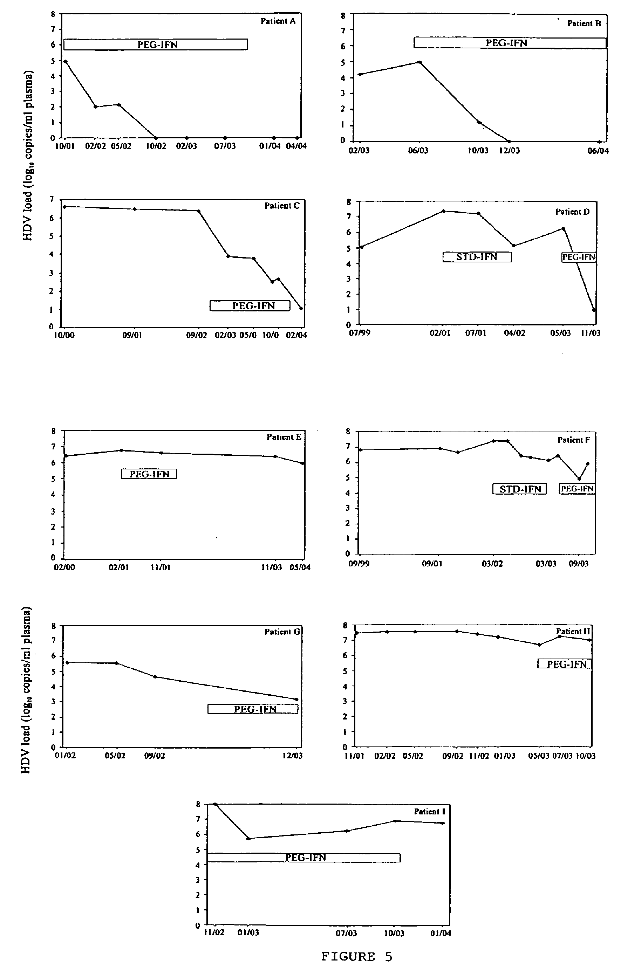

- Figure 5 illustrates a HDV-RNA quantification in chronically infected patients:
   A: Patients displaying a "responder" profile: patients A and B responded to the IFN treatment and negativated their viral load. Patients C and D remained detectable at the end of the treatment but the viral load showed a decrease of more than 5 log₁₀ copies/ml plasma.
   B: Patients displaying a "non-responder" profile: viral load decrease did not exceed 2 log₁₀ copies/ml during the course of the treatment. In the case of patients E, F, H and I, the initial viral load was superior to 6 log₁₀ copies/ml.
   C: Patient J: HDV super-infection in a HBV-HIV infected patient. Patient K: liver transplantation after 2 treatment failures.

### Example 1: MATERIAL AND METHODS

### 1.1 Patients and samples

Eighty four blood samples were used for the technical validation of the assay (Table 1).

**TABLE 1: Samples used for the technical validation of the assay**

| | Sample number | Characteristics |
|---|---|---|
| | 1 to 5 | HBsAg positive |
| | 6 to 11 | HCV-RNA positive |
| Anti-HDV negative samples | 12 to 15 | anti-HAV IgG positive |
| | 16 to 20 | HIV-RNA positive |
| | 21 | HEV-RNA positive |
| | 22 to 26 | negative for viral hepatitis markers^{a} |
| | 27 to 33 | Intra-assay reproducibility |
| | 34 to 37 | Inter-assay reproducibility |
| | 38 to 40 | Assay sensitivity |
| | 41 to 63 | HDV-1 |
| Anti-HDV positive samples | 64 | HDV-2 |
| | 65 to 70 | HDV-5 |
| | 71 to 74 | HDV-6 |
| | 75 to 79 | HDV-7 |
| | 80 to 84 | HDV-3 |
| ^{a}Anti-HDV, HbsAg, HCV-RNA, anti-HAV IgG, HIV-RNA and HEV-RNA | | |

Samples 1 to 26, negative for HDV serology, were tested to evaluate the assay specificity. Among these samples, 5 were positive for HBs Antigen and HBV DNA, and 16 were negative for HBs Antigen screening but positive either for Hepatitis C virus (HCV) RNA (n=6), or Hepatitis A virus (HAV) IgM (n=4), Human immunodeficiency virus (HIV) RNA (viral load > 10,000 copy/ml, n=5), Hepatitis E virus RNA (n=1). The remaining 5 were negative for HCV-RNA, HAV-IgM and HIV-RNA. Samples 41 to 79, corresponding to HDV-1 (n=23), HDV-2 (n=1), HDV-5 (n=6), HDV-6 (n=4), and HDV-7 (n=5) (according to Radjef et al. ²) were tested both with a qualitative RT-PCR assay, and the quantitative real-time RT-PCR assay, in order to evaluate the ability of the real-time RT-PCR assay to detect and quantify the genome of any HDV type. HDV-3 samples (80 to 84) were available from a collection of leftover dried blood spot filter paper specimens collected by means of fingertip prick in the Amazonia region, among infected local people. The filter papers were air-dried and stored at room temperature until use. For testing, 7 mm disks were punched out of the center of each dried blood spot and placed into 500 µl of 9%o NaCl solution at -20°C. RNA was extracted from 250 µl eluates using the QIAamp MinElute Virus Vacuum (QIAGEN) according to the manufacturer's recommendations.

Total HDV-infected woodchuck-5 liver RNA (infected with HDV-1 prototype³⁰) was used as an external control for the real-time quantitative RT-PCR ³¹.

Eleven patients, labeled A to K, (76 samples) receiving IFN (or its pegylated form: PEG-IFN) for chronic HDV infection, and for whom sequential plasmatic samples had been kept frozen at -80°C, were selected for a retrospective HDV-RNA quantification. These samples had previously been tested with a qualitative assay designed to amplify the *R0* region of the HDV genome as defined in the International PCT Application WO 03/0227291 (nucleotides 889 to 1289) (Figure 1) ³². Age, sex, HDV type and HIV status of these patients are presented in Table 2.

**TABLE 2: Clinical features of the patients selected for a retrospective sequential HDV-RNA Quantification in blood samples**

| | Sex | age | HDV clade | HIV status | Number of samples tested |
|---|---|---|---|---|---|
| Patient A | M | 54 | 1*^{a}* | NEG^{c} | 7 |
| Patient B | M | 40 | 1*^{b}* | NEG | 4 |
| Patient C | F | 45 | 1*^{a}* | NEG | 8 |
| Patient D | M | 37 | 1*^{a}* | NEG | 6 |
| Patient E | M | 52 | 1*^{a}* | NEG | 4 |
| Patient F | M | 44 | 1*^{b}* | NEG | 11 |
| Patient G | M | 42 | 1*^{a}* | NEG | 4 |
| Patient H | M | 37 | 1*^{b}* | POS^{c} | 9 |
| Patient I | M | 43 | 1*^{b}* | POS | 4 |
| Patient J | M | 54 | 1*^{b}* | POS | 10 |
| Patient K | M | 40 | 5*^{b}* | POS | 9 |
| *^{a}* Genotype as determined with RFLP | | | | | |
| *^{b}* Genotype as determined after sequencing of the *RO* region | | | | | |
| *^{c}* NEG: negative HIV serology, POS: positive HIV serology | | | | | |

### 1.2 Real-time PCR quantification in serum samples

HDV-RNA was extracted from 250 µl serum or plasma using the QIAamp MinElute Virus Vacuum (QIAGEN). cDNA synthesis was performed with 5 µl of the extraction product, in a 25 µl mixture containing 0.5 mmol/l GeneAmp dNTPs (Applied Biosystems), 0.4 pmol/µl Random Hexamers (Applied Biosystems), 1 IU/µl RNasin (Promega), 100 IU/µl Superscript II RNase H Reverse Transcriptase (Invitrogen), 1X First-Strand Buffer (Invitrogen), and 10 mmol/µl DTT (Invitrogen). The reaction was performed for 45 min at 42°C. cDNA was purified using Montage PCR Centrifugal Filter Devices (Millipore).

cDNA is purified on Microcon 50 columns.

Primers and probe were designed using Primer Express® Software v2.0 (Applied Biosystems) with slight modifications taking into account the genetic variability of HDV ². The forward primer was selected in the ribozyme region of the genome, and the reverse primer in the region I of the anti-genome ribozyme. The probe, which was defined in the same region as the reverse primer, was designed to anneal in the anti-genomic sequence to avoid pairing to the reverse primer (Figure 2). The name and sequence of the primers and probe were: Delta-F (direct primer): 5'-GCATGGTCCCAGCCTCC-3' SEQ ID NO:1), Delta-R (reverse primer): 5'-TCTTCGGGTCGGCATGG-3' (SEQ ID NO:2), Delta-P (probe): 5'-FAM-ATGCCCAGGTCGGAC-MGB-3' (SEQ ID NO:4).

Because of the existence of one mismatch with the sequences of type III genome, a second direct primer was specifically designed for the amplification of HDV-3 isolates: T3-Delta-F: 5'-GCATGGCCCCAGCCTCC-3' (SEQ ID NO:3).

The real-time PCR reactions were carried out using the TaqMan® Universal PCR Master Mix (Applied Biosystems). Purified cDNA (10 µl) was added to a 40 µl PCR mixture containing 25 µl TaqMan® Universal PCR Master Mix, 0.3 mmol/µl of each primer and 0.2 mmol/µl fluorogenic probe. The reaction consisted of one initiating step of 2 min at 50°C, followed by 10 min at 95°C, and 45 cycles of amplification including 15 sec at 95°C and 1 min at 60°C. The reaction, data acquisition and analysis were performed using the ABI PRISM 7000 Sequence Detection System (Applied Biosystems). The number of target copies in the reaction was deduced from the threshold cycle (*C_{T}*) values corresponding to the fractional cycle number at which the released fluorescence exceeded 20 times the standard deviation of the mean baseline emission.

The plasmid (pCRII-dFr45-*R1* ²), containing one copy of the *R'1* region of the HDV genome as defined in the International PCT Application WO 03/027291 (nucleotides 305 to 1161 in reference to Wang *et al* ³) (Figure 1) was used as a standard for HDV-cDNA quantification. After *EcoRI* (Biolabs) digestion of the plasmid, the *R'1* region was purified from an agarose gel using QIAEX II (Qiagen). The concentration of purified *R'1* DNA was determined with a multichannel spectrophotometer and the corresponding copy number was calculated.

### 1.3 Qualitative RT-PCR for HDV-RNA detection in serum samples

RNA extraction and cDNA synthesis were performed as described for the quantitative assay. *R0* region of the genome, in reference to International PCT Application WO 03/027291 (nucleotides 889 to 1289) was amplified as described in said International PCT Application using primers 900s and 1280as as previously described ³² (Figure 1). Amplicons were detected after electrophoresis in a 1.5% agarose gel stained with ethidium bromide.

HDV type determination was performed from the amplified *R0* region, using either a 2-steps RFLP procedure as described in WO 03/027291 or a previously described phylogenetic analysis of the sequence².

### Statistical analysis

Mann Whitney non-parametric U test (StatView 4.02) was used for statistical comparisons. Differences were considered significant at *P*≤0.05.

### Example 2: Specificity, sensitivity, and reproducibility of the real-time PCR assay for the detection and quantification of HDV-RNA in serum.

The assay specificity was ascertained by the negative results obtained with a panel of 26 control serum samples after 45 cycles of amplification (results not shown).

The HDV-*R*'*1* fragment was used as a standard to quantify HDV-RNA. Ten-fold serial dilutions ranging from 10 to 10⁷ copies were tested in triplicate, plotting the mean *C_{T}* values against the copy number in order to construct the standard curve. The correlation coefficient was repeatedly superior to 0.997 and the slope was - 3.5. The amplification efficiency, calculated as [10^{(-1/slope)}-1] X 100, was 93%. The dilution corresponding to an input of 10 copies per reaction was repeatedly detected with a 100% sensitivity (results not shown). Thus, according to the dilution factors during the RNA extraction and RT procedures, the sensitivity of the assay for quantification of clinical samples was approximately 1000 copies/ml of the target sequence. Samples for which the real-time RT-PCR assay detected from 1 to 1000 copies/ml plasma, were considered positive for HDV-RNA, though below the quantification limit.

The intra-assay reproducibility was evaluated by 2 approaches. First, 8 replicates of 10-fold standard dilutions ranging from 10 to 10⁷ copies per reaction were tested in the same experiment. The coefficients of variation (CV) of the *C_{T}* ranged from 0.4% to 2.6% and are reported in Table 3.

**TABLE 3: Intra-assay reproducibility for the amplification of the plasmid standard**

| | Input genome equivalent of plasmid standard | | | | | | |
|---|---|---|---|---|---|---|---|
| | 10 | 100 | 1,000 | 10,000 | 100,000 | 1,000,000 | 10,000,000 |
| Mean C*_{T}* values | 38.74 | 32.75 | 29.66 | 26.04 | 22.57 | 19.21 | 16.92 |
| CV% | 2.6 | 1.6 | 1.4 | 0.5 | 0.7 | 0.4 | 0.6 |

Second, 3 replicates of 7 serum samples (numbered 27 to 33 - Table 1) were independently submitted to RNA extraction, cDNA synthesis and real-time PCR in the same experiment. The mean viral load of the samples ranged from 70 to 21,500 copies and the CV ranged from 1.8% to 25% (Table 4).

**TABLE 4: Intra-assay reproducibility for serum samples extracted independently**

| | Sample number | | | | | | |
|---|---|---|---|---|---|---|---|
| | 27 | 28 | 29 | 30 | 31 | 32 | 33 |
| mean copy number (/10µl) | 21,300 | 4,700 | 3,200 | 2,100 | 750 | 130 | 70 |
| CV% | 2.7 | 7.4 | 1.8 | 7.2 | 7.7 | 25 | 7.1 |

To estimate the inter-assay variability, RNA was extracted from 4 serum samples (numbered 34 to 37), 3 times, by 3 different operators, on 3 distinct days. The mean viral load of the samples ranged from 17 to 2,200 copies, and the CV ranged from 3.3% to 25.4% (Table 5).

**TABLE 5: Inter-assay reproducibility**

| | Sample number | | | |
|---|---|---|---|---|
| | 34 | 35 | 36 | 37 |
| mean copy number (/10µl) | 2,200 | 1,300 | 880 | 17 |
| CV% | 25.4 | 24.1 | 3.3 | 17.3 |

The real-time PCR assay and the qualitative assay were compared in terms of sensitivity. Three cDNA obtained from 3 distinct serum samples (numbered 38 to 40) were diluted in order to determine the end point dilution value for each assay. Purified cDNA were diluted as follows: 1/2, 1/5, 1/10, 1/25, 1/50, 1/100, 1/250, 1/500, 1/1,000, 1/5,000 and 1/10,000, and each dilution was tested in parallel with both qualitative and quantitative assays. With the qualitative assay, the last visible dilution after ethidium bromide staining of the agarose gel was 1/100 for sample 38, and 1/1,000 for samples 39 and 40. With the real-time PCR assay the end point dilutions were 1/500, 1/1,000, and 1/5,000 for samples 38, 39 and 40 respectively, indicating that the quantitative assay was at least as sensitive as the qualitative assay (Figure 3).

### Example 3: HDV-RNA quantification in blood samples

The ability of the assay to quantify HDV-RNA of different clades in serum was verified by the detection of HDV-RNA in 44 serum samples numbered 41 to 84 (Figure 4). No significant difference was noted between the range of viral loads obtained with HDV-1 viruses and viruses of other types, indicating that the assay was adapted to detect and quantify any HDV. Twenty three HDV-1 samples, numbered 41 to 63 were tested and the median copy number was 1,950, ranging from 2 to 986,000 copies /10 µl cDNA. Serum samples containing HDV-2 (n=1), 5 (n=6), 6 (n=4), and 7 (n=5) were also tested, and the median viral load was 1,300, ranging from 2 to 1,000,000 copies /10 µl cDNA. The same samples were tested with the qualitative assay, and the intensity of the ethidium bromide stained bands appeared identical for all samples with a viral loads ≥ 500 copies/10 µl cDNA (results not shown).

The ability of the assay to detect HDV-3 RNA was evaluated using samples 80 to 84, which had been obtained after elution of dried blood spots, and found positive for HDV-RNA with the qualitative assay (although only a faint signal was detected on the ethidium bromide stained agarose gel for samples 82 to 84- Table 6).

| TABLE 6: HDV-3 RT-PCR results | | | |
|---|---|---|---|
| | Qualitative RT-PCR^{a} | Real-time RT-PCR C_{T} values | |
| | | Using primer Delta-F | using primer T3-Delta-F |
| Sample 80 | ++ | 44.55 | 35.38 |
| Sample 81 | + | 39.54 | 32.84 |
| Sample 82 | ± | >45*^{b}* | 41.28 |
| Sample 83 | ± | >45 | 36.56 |
| Sample 84 | ± | >45 | >45*^{b}* |
| ^{a} Visual appreciation of the BET signal intensity: ±, faintly visible band; +, band of low intensity; | | | |
| ⁺⁺, band of medium intensity | | | |
| *^{b}* C_{T} values (>45 indicates a negative result) | | | |

Considering the nature of these samples, the quantitative results obtained with the real-time RT-PCR assay were not taken into account. HDV-RNA from samples 80 and 81 was detected using either Delta-F (SEQ ID NO:1) or T3Delta-F (SEQ ID NO:3) as forward primers. For samples 82 and 83, HDV-RNA was detected with primer T3Delta-F (SEQ ID NO:3) only, and sample 84 remained negative in both conditions (Table 6). However, one could expect that viral RNA obtained directly from plasma samples might give better results.

### Example 4: HDV-RNA quantification in chronically infected patients

The real-time RT-PCR assay was evaluated as a means to follow the evolution of HDV viral load in the plasma of infected patients receiving IFN for chronic delta hepatitis. Eleven patients, for whom serial samples previously tested in the laboratory with the qualitative RT-PCR were available, were considered for this follow up. The viral loads, expressed as log₁₀ copy number per ml plasma are reported in Figure 5.

Three groups of patients were distinguished. In the first group (Figure 5A), the evolution of the viral loads indicated a favorable answer to IFN therapy, with a diminution of at least 5 log₁₀/ml during the course of the treatment. For patients A and B, HDV-RNA was found undetectable with both qualitative and quantitative assays at the end of the treatment. Patient C, who had received PEG-IFN from December 2002 to December 2003, presented a viral load which was dramatically reduced, but still detectable 2 months after the end of the treatment, and patient D, who had not responded to a previous IFN therapy, responded to PEG-IFN (with a loss of 5 log₁₀ copies per ml). In these two cases, HDV RNA remained detectable with the qualitative assay, with no indication of a virological response.

In the second group of patients, (Figure 5B) the reduction of the viral load under PEG-IFN therapy did not exceed 2.5 log₁₀ copy/ml. Patients E, F, H and I, presented viral loads over 6.5 log₁₀ copies/ml at the initiation of therapy, and patients H and I were co-infected with HIV (Table 2).

The cases of patients J and K should be interpreted individually (Figure 5C). Patient J, who was chronically infected with HBV, had been negative for HDV-markers until September 2001, indicating that a super-infection with HDV had occurred between June 2000 and September 2001. In December 2001, viral RNA was undetectable, suggesting spontaneous virus neutralization. However, in October 2002, the viral load was back to 5.5 log₁₀ copy/ml. A treatment with PEG-IFN was then initiated in February 2003, leading to a rapid reduction of the viral load, which has remained undetectable ever since, with both quantitative and qualitative assays. Patient K, who had unsuccessfully received STD-IFN in 1999, developed an hepatic carcinoma in 2001, while being treated. This patient underwent hepatic transplantation in May 2002, and his viral load has remained undetectable since.

The eleven patients chronically infected with HDV, and for whom sequential plasma samples were available, were evaluated in terms of HDV viral load. Two patterns of virological response to IFN were distinguished. In the first group, a significant decrease of the viral load (at least 5 log₁₀/ml) was observed during the course of the treatment, allowing classifying patients A and B as "responders" to one-year PEG-IFN therapy. For patient C, the qualitative RT-PCR performed in the laboratory during the course of the treatment, had remained constantly positive, giving no evidence of a virological response. However, when considering the quantitative results of the PCR, the patient obviously responded to the treatment (with a decrease of 5.5 log₁₀/ml). The treatment was ended after one year, but would have probably been pursued if the quantitative results had been available. In the case of patient D, a significant decrease of the HDV-RNA load was observed during the course of PEG-IFN therapy, although the patient had remained resistant to a previous one-year treatment with IFN. This might indicate a better efficacy of PEG-IFN for the treatment of chronic delta hepatitis, and the quantitative RT-PCR assay will be useful to evaluate this point. Patients E to I remained "non responders" to PEG-IFN, indicating that other factors might interfere with the treatment success. For example, the initial viral load was globally higher in the non-responder group (P=0.05), and this could indicate that the baseline viral load might predict treatment efficacy and could be taken into account in the management of the treatment, in terms of dose and duration.

### REFERENCES

1. Gerin JL, Casey JL, Purcell RH. Hepatitis Delta Virus. In: Hollinger FB, Purcell RH, Gerin JL, eds. Viral hepatitis. Philadelphia: Lippincott Williams and Wilkins; 2002:169-182
2. Radjef N, Gordien E, Ivaniushina V, Gault E, Anais P, Drugan T, Trinchet JC, Roulot D, Tamby M, Milinkovitch MC, Deny P. Molecular Phylogenetic Analyses Indicate a Wide and Ancient Radiation of African Hepatitis Delta Virus, Suggesting a Deltavirus Genus of at Least Seven Major Clades. J Virol. 2004;78:2537-2544.
3. Wang KS, Choo QL, Weiner AJ, Ou JH, Najarian RC, Thayer RM, Mullenbach GT, Denniston KJ, Gerin JL, Houghton M. Structure, sequence and expression of the hepatitis delta (delta) viral genome. Nature. 1986;323:508-514.
4. Casey JL, Brown TL, Colan EJ, Wignall FS, Gerin JL. A genotype of hepatitis D virus that occurs in northern South America. Proc Natl Acad Sci U S A. 1993;90:9016-9020.
5. Imazeki F, Omata M, Ohto M. Complete nucleotide sequence of hepatitis delta virus RNA in Japan. Nucleic Acids Res. 1991;19:5439.
6. Shakil AO, Hadziyannis S, Hoofnagle JH, Di Bisceglie AM, Gerin JL, Casey JL. Geographic distribution and genetic variability of hepatitis delta virus genotype I. Virology. 1997;234:160-167.
7. Wu JC, Chiang TY, Sheen IJ. Characterization and phylogenetic analysis of a novel hepatitis D virus strain discovered by restriction fragment length polymorphism analysis. J Gen Virol. 1998;79:1105-1113.
8. Farci P. Delta hepatitis: an update. J Hepatol. 2003;39:5212-S219
9. Rizzetto M, Verme G, Recchia S, Bonino F, Farci P, Arico S, Calzia R, Picciotto A, Colombo M, Popper H. Chronic hepatitis in carriers of hepatitis B surface antigen, with intrahepatic expression of the delta antigen. An active and progressive disease unresponsive to immunosuppressive treatment. Ann Intern Med. 1983;98:437-441.
10. Fattovich G, Giustina G, Christensen E, Pantalena M, Zagni I, Realdi G, Schalm SW. Influence of hepatitis delta virus infection on morbidity and mortality in compensated cirrhosis type B. The European Concerted Action on Viral Hepatitis (Eurohep). Gut. 2000;46:420-426.
11. Saracco G, Rosina F, Brunetto MR, Amoroso P, Caredda F, Farci P, Piantino P, Bonino F, Rizzetto M. Rapidly progressive HBsAg-positive hepatitis in Italy. The role of hepatitis delta virus infection. J Hepatol. 1987;5:274-281.
12. Farci P, Mandas A, Coiana A, Lai ME, Desmet V, Van Eyken P, Gibo Y, Caruso L, Scaccabarozzi S, Criscuolo D, et al. Treatment of chronic hepatitis D with interferon alfa-2a. N Engl J Med. 1994;330:88-94.
13. Rosina F, Pintus C, Meschievitz C, Rizzetto M. A randomized controlled trial of a 12-month course of recombinant human interferon-alpha in chronic delta (type D) hepatitis: a multicenter Italian study. Hepatology. 1991;13:1052-1056.
14. Farci P, Roskams T, Chessa L, Peddis G, Mazzoleni AP, Scioscia R, Serra G, Lai ME, Loy M, Caruso L, Desmet V, Purcell RH, Balestrieri A. Long-term benefit of interferon alpha therapy of chronic hepatitis D: regression of advanced hepatic fibrosis. Gastroenterology. 2004;126:1740-1749.
15. Lau DT, Doo E, Park Y, Kleiner DE, Schmid P, Kuhns MC, Hoofnagle JH. Lamivudine for chronic delta hepatitis. Hepatology. 1999;30:546-549.
16. Wolters LM, van Nunen AB, Honkoop P, Vossen AC, Niesters HG, Zondervan PE, de Man RA. Lamivudine-high dose interferon combination therapy for chronic hepatitis B patients co-infected with the hepatitis D virus. J Viral Hepat. 2000;7:428-434.
17. Berk L, de Man RA, Housset C, Berthelot P, Schalm SW. Alpha lymphoblastoid interferon and acyclovir for chronic hepatitis delta. Prog Clin Biol Res. 1991;364:411-420.
18. Garripoli A, Di Marco V, Cozzolongo R, Costa C, Smedile A, Fabiano A, Bonino F, Rizzetto M, Verme G, Craxi A, et al. Ribavirin treatment for chronic hepatitis D: a pilot study. Liver. 1994;14:154-157.
19. Yurdaydin C, Bozkaya H, Gurel S, Tillmann HL, Aslan N, Okcu-Heper A, Erden E, Yalcin K, Iliman N, Uzunalimoglu O, Manns MP, Bozdayi AM. Famciclovir treatment of chronic delta hepatitis. J Hepatol. 2002;37:266-271.
20. Deny P, Lecot C, Jeantils V, Ovaguimian L, Krivitzky A, Brechot C. Polymerase chain reaction-based detection of hepatitis D virus genome in patients infected with human immunodeficiency virus. J Med Virol. 1993;39:214-218.
21. Madejon A, Cotonat T, Bartolome J, Castillo I, Carreno V. Treatment of chronic hepatitis D virus infection with low and high doses of interferon-alpha 2a: utility of polymerase chain reaction in monitoring antiviral response. Hepatology. 1994;19:1331-1336.
22. Goudeau A, Dubois F, Leturcq P. [Delta hepatitis]. Presse Med. 1987; 16:2117-2122.
23. Jardi R, Buti M, Cotrina M, Rodriguez F, Allende H, Esteban R, Guardia J. Determination of hepatitis delta virus RNA by polymerase chain reaction in acute and chronic delta infection. Hepatology. 1995;21:25-29.
24. Wu JC, Lee SD, Govindarajan S, Kung TW, Tsai YT, Lo KJ, Ting LP. Correlation of serum delta RNA with clinical course of acute hepatitis delta virus superinfection in Taiwan: a longitudinal study. J Infect Dis. 1990;161:1116-1120.
25. Deny P, Fattovich G, Le Gal F, Giustina G, Lecot C, Morsica G, Poinsot H, Alberti A, Brechot C. Polymerase-chain-reaction-based semi-quantification of hepatitis D viraemia in patients treated with high doses of alpha 2b interferon. Res Virol. 1994;145:287-295.
26. Gault E, Michel Y, Dehee A, Belabani C, Nicolas JC, Garbarg-Chenon A. Quantification of human cytomegalovirus DNA by real-time PCR. J Clin Microbiol. 2001;39:772-775.
27. Gourlain K, Salmon D, Gault E, Leport C, Katlama C, Matheron S, Costagliola D, Mazeron MC, Fillet AM. Quantitation of cytomegalovirus (CMV) DNA by real-time PCR for occurrence of CMV disease in HIV-infected patients receiving highly active antiretroviral therapy. J Med Virol. 2003;69:401-407.
28. Takeuchi T, Katsume A, Tanaka T, Abe A, Inoue K, Tsukiyama-Kohara K, Kawaguchi R, Tanaka S, Kohara M. Real-time detection system for quantification of hepatitis C virus genome. Gastroenterology. 1999;116:636-642.
29. Yamshiro T, Nagayama K, Enomoto N, Watanabe H, Miyagi T, Nakasone H, Sakugawa H, Watanabe M. Quantification of the level of hepatitis delta virus RNA in serum, by real-time polymerase chain reaction - and its possible correlation with the clinical stage of liver disease. J Infect Dis. 2004;189:1151-1157
30. Ponzetto A, Cote PJ, Popper H, Hoyer B, London WT, Ford E, Ferruccio B, Purcell R, Gerin J. Transmission of the hepatitis B virus-associated delta agent to the eastern woodchuck. Proc Natl Acad Sci U S A. 1984;81:2208-2212
31. Deny P, Zignego AL, Rascalou N, Ponzetto A, Tiollais P, Brechot C. Nucleotide sequence analysis of three different hepatitis delta viruses isolated from a woodchuck and humans. J Gen Virol. 1991;72:735-739.
32. Ivaniushina V, Radjef N, Alexeeva M, Gault E, Semenov S, Salhi M, Kiselev O, Deny P. Hepatitis delta virus genotypes I and II cocirculate in an endemic area of Yakutia, Russia. J Gen Virol. 2001;82:2709-2718.
33. Manns MP, McHutchison JG, Gordon SC, Rustgi VK, Shiffman M, Reindollar R, Goodman ZD, Koury K, Ling M, Albrecht JK. Peginterferon alfa-2b plus ribavirin compared with interferon alfa-2b plus ribavirin for initial treatment of chronic hepatitis C: a randomised trial. Lancet. 2001;358:958-965.
34. Smedile A, Rizzetto M, Denniston K, Bonino F, Wells F, Verme G, Consolo F, Hoyer B, Purcell RH, Gerin JL. Type D hepatitis: the clinical significance of hepatitis D virus RNA in serum as detected by a hybridization-based assay. Hepatology. 1986;6:1297-1302.
35. Rasshofer R, Buti M, Esteban R, Jardi R, Roggendorf M. Demonstration of hepatitis D virus RNA in patients with chronic hepatitis. J Infect Dis. 1988;157:191-195.
36. Bonino F, Heermann KH, Rizzetto M, Gerlich WH. Hepatitis delta virus: protein composition of delta antigen and its hepatitis B virus-derived envelope. J Virol. 1986;58:945-950.
37. Kos A, Dijkema R, Arnberg AC, van der Meide PH, Schellekens H. The hepatitis delta (delta) virus possesses a circular RNA. Nature. 1986;323:558-560.
38. Modahl LE, Lai MM. Hepatitis delta virus: the molecular basis of laboratory diagnosis. Crit Rev Clin Lab Sci. 2000;37:45-92.
39. Branch AD, Polaskova JA, Schreiber DR. Tm studies of a tertiary structure from the human hepatitis delta agent which functions in vitro as a ribozyme control element. Nucleic Acids Res. 1995;23:4391-4399.
40. Chao YC, Chang MF, Gust I, Lai MM. Sequence conservation and divergence of hepatitis delta virus RNA. Virology. 1990; 178:384-392.
41. Perrotta AT, Been MD. A pseudoknot-like structure required for efficient self-cleavage of hepatitis delta virus RNA. Nature. 1991;350:434-436.
42. Livak K et al., Guidelines for designing TaqMan^{™} fluorogenic probes for 5' nuclease assays.
43. Poitras et al., Reviews in Biology and Biotechnology, 2002, 2, 1-11.
44. Gibson UEM et al., Genome Research, 1996, 6, 995-1001.

## Claims

1. Method for detecting HDV-RNA in a biological sample, which method is **characterized in that** it comprises:
(1) a step of extracting HDV-RNA from a biological sample and
(2) a step of evaluating the quantity of said HDV-RNA by real-time RT-PCR quantification comprising:
- amplifying the cDNA corresponding to said HDV-RNA in the presence of at least a pair of primers selected from the group consisting of the following pairs:
(a) Delta-F (direct primer): 5'-GCATGGTCCCAGCCTCC-3'(SEQ ID NO:1) and Delta-R (reverse primer): 5'-TCTTCGGGTCGGCATGG-3' (SEQ ID NO:2) and
(b) T3-Delta-F (direct primer): 5'-GCATGGCCCCAGCCTCC-3' (SEQ ID NO:3) and Delta-R (reverse primer): 5'-TCTTCGGGTCGGCATGG-3' (SEQ ID NO:2); and
- detecting the quantity of the formed amplicons and comparing said quantity with at least a control sample.

2. Method according to claim 1, **characterized in that** the detecting is performed in the presence of a fluorogenic probe [Delta-P (probe)] consisting of the following sequence: 5'- ATGCCCAGGTCGGAC-3' (SEQ ID NO:4).

3. Method according to claims 1 or 2, **characterized in that** said detecting step includes as control sample, positive control sample selected in the group consisting of R' 1 region of an HDV genome, corresponding to positions 305-1161 of said genome, a fragment of said region R' 1 comprising positions 692-904 of aid genome and the whole genome of an HDV.

4. Method according to claims 1 or 2, **characterized in that** said detecting step includes as control sample, a positive control sample as defined in claim 3 and a negative control.

5. Method according to any of claims 1 to 4, **characterized in that** said fluorogenic probe is labeled at least at one of its ends by a fluorescent dye.

6. A detection kit, **characterized in that** it comprises further to the buffers and the reagents necessary for extracting HDV-RNA from a biological sample, synthetizing its corresponding cDNA and amplifying said cDNA, at least two pairs of primers as defined in claim 1.

7. Detection kit according to claim 6, **characterized in that** it further comprises:
- a fluorogenic probe, as defined in claim 2,
- a positive control sample as defined in claim 3 and
- a negative control.

8. Detection kit according to claim 7, **characterized in that** said fluorogenic probe is labeled at its 5' end with a fluorescent reporter dye and at its 3' end with a quencher dye and a MGB group.

9. Pair of primers able to amplify any HDV genome, said pair of primers being selected in the group consisting of the pair of primers SEQ ID NO:1 and SEQ ID NO:2 and the pair of primers SEQ ID NO:3 and SEQ ID NO:2.

10. Probe specific of the nucleic acid fragment amplified by one pair of primers as defined in claim 9, said probe consisting of the sequence defined by SEQ ID NO:4

## Patentansprüche

1. Verfahren zum Detektieren von HDV-RNA in einer biologischen Probe, wobei das Verfahren **dadurch gekennzeichnet ist, dass** es umfasst:
(1) eine Stufe des Extrahierens von HDV-RNA aus einer biologischen Probe und
(2) eine Stufe des Bewertens der Menge von HDV-RNA mittels Real-Time-RT-PCR-Quantifizierung, umfassend:
- Amplifizieren der cDNA, entsprechend der HDV-RNA, in Gegenwart von mindestens einem Primerpaar, ausgewählt aus der Gruppe, bestehend aus den folgenden Paaren:
(a) Delta-F (direkter Primer): 5'-GCATGGTCCCAGCCTCC-3' (SEQ ID NO: 1) und Delta-R (Rückwärtsprimer): 5'-TCTTCGGGTCGGCATGG-3' (SEQ ID NO: 2) und
(b) T3-Delta-F (direkter Primer): 5'-GCATGGCCCCAGCCTCC-3' (SEQ ID NO: 3) und Delta-R (Rückwärtsprimer): 5'-TCTTCGGGTCGGCATGG-3' (SEQ ID NO: 2); und
- Detektieren der Menge der gebildeten Amplikons und Vergleichen der Menge mit mindestens einer Kontrollprobe.

2. Verfahren gemäß Anspruch 1, **dadurch gekennzeichnet, dass** das Detektieren in Gegenwart einer fluorogenen Sonde [Delta-P (Sonde)], bestehend aus der folgenden Sequenz: 5*'-*ATGCCCAGGTCGGAC-3' (SEQ ID NO: 4), durchgeführt wird.

3. Verfahren gemäß den Ansprüchen 1 oder 2, **dadurch gekennzeichnet, dass** die Detektionsstufe als Kontrollprobe eine Positiv-Kontrollprobe einschließt, ausgewählt aus der Gruppe, bestehend aus der R'1-Region eines HDV-Genoms, entsprechend den Positionen 305-1161 des Genoms, einem Fragment der Region R*'*1, umfassend die Positionen 692-904 des Genoms, und dem gesamten Genom eines HDV.

4. Verfahren gemäß den Ansprüchen 1 oder 2, **dadurch gekennzeichnet, dass** die Detektionsstufe als Kontrollprobe eine Positiv-Kontrollprobe, wie in Anspruch 3 definiert, und eine Negativkontrolle einschließt.

5. Verfahren gemäß einem der Ansprüche 1 bis 4, **dadurch gekennzeichnet, dass** die fluorogene Sonde an mindestens einem ihrer Enden mit einem Fluoreszenzfarbstoff markiert ist.

6. Detektionskit, **dadurch gekennzeichnet, dass** er zu den Puffern und Reagenzien, die für das Extrahieren von HDV-RNA aus einer biologischen Probe, das Synthetisieren ihrer entsprechenden cDNA und das Amplifizieren der cDNA notwendig sind, weiterhin mindestens zwei Primerpaare, wie in Anspruch 1 definiert, umfasst.

7. Detektionskit gemäß Anspruch 6, **dadurch gekennzeichnet, dass** er weiterhin umfasst:
- eine fluorogene Sonde, wie in Anspruch 2 definiert,
- eine Positiv-Kontrollprobe, wie in Anspruch 3 definiert, und
- eine Negativkontrolle.

8. Detektionskit gemäß Anspruch 7, **dadurch gekennzeichnet, dass** die fluorogene Sonde an ihrem 5'-Ende mit einem fluoreszierenden Reporterfarbstoff und an ihrem 3*'*-Ende mit einem Quencher-Farbstoff und einer MGB-Gruppe markiert ist.

9. Primerpaar, das in der Lage ist, jegliches HDV-Genom zu amplifizieren, wobei das Primerpaar ausgewählt ist aus der Gruppe, bestehend aus dem Primerpaar SEQ ID NO: 1 und SEQ ID NO: 2 und dem Primerpaar SEQ ID NO: 3 und SEQ ID NO: 2.

10. Sonde, spezifisch für das Nucleinsäurefragment, amplifiziert mittels eines Primerpaares, wie in Anspruch 9 definiert, wobei die Sonde aus der Sequenz besteht, die durch SEQ ID NO: 4 definiert ist.

## Revendications

1. Procédé pour détecter de l'ARN de VHD dans un échantillon biologique, lequel procédé est **caractérisé en ce qu'**il comprend :
(1) une étape d'extraction de l'ARN de VHD à partir d'un échantillon biologique et
(2) une étape d'évaluation de la quantité dudit ARN de VHD via une quantification par RT-PCR en temps réel, comprenant les étapes consistant à :
- amplifier l'ADNc correspondant au dit ARN de VHD en présence d'au moins une paire d'amorces, sélectionnée dans le groupe constitué par les paires suivantes :
(a) Delta-F (amorce directe): 5'-GCATGGTCCCAGCCTCC-3' (SEQ ID n°:1) et Delta-R (amorce inverse): 5'-TCTTCGGGTCGGCATGG-3' (SEQ ID n°:2) et
(b) T3-Delta-F (amorce directe): 5'-GCATGGCCCCAGCCTCC-3' (SEQ ID n°:3) et Delta-R (amorce inverse): 5'-TCTTCGGGTCGGCATGG-3' (SEQ ID n°:2); et
- détecter la quantité d'amplicons formés, ainsi que comparer ladite quantité avec au moins un échantillon témoin.

2. Procédé, selon la revendication 1, **caractérisé en ce que** la détection est effectuée en présence d'une sonde fluorogène [Delta-P (sonde)] consistant en la séquence suivante: 5'-ATGCCCAGGTCGGAC-3' (SEQ ID n°:4).

3. Procédé, selon les revendications 1 ou 2, **caractérisé en ce que** ladite étape de détection comprend, comme échantillon témoin, un échantillon témoin positif sélectionné dans le groupe consistant en la région R'1 du génome d'un VHD, correspondant aux positions 305 à 1161 dudit génome, d'un fragment de ladite région R' 1, comprenant les positions 692 à 904 dudit génome, et le génome complet d'un VHD.

4. Procédé, selon les revendications 1 ou 2, **caractérisé en ce que** ladite étape de détection comprend, comme échantillon témoin, un échantillon témoin positif, tel qu'il est défini à la revendication 3, et un témoin négatif.

5. Procédé, selon l'une quelconque des revendications 1 à 4, **caractérisé en ce que** ladite sonde fluorogène est marquée au moins à l'une de ses extrémités avec un fluorochrome.

6. Kit de détection **caractérisé en ce qu'**il comprend, en plus des tampons et des réactifs nécessaires à une extraction de l'ARN d'un VHD provenant d'un échantillon biologique, à une synthèse de son ADNc correspondant et à l'amplification dudit ADNc, au moins deux paires d'amorces, telles qu'elles sont définies à la revendication 1.

7. Kit de détection, selon la revendication 6, **caractérisé en ce qu'**il comprend en outre:
- une sonde fluorogène, telle qu'elle est définie à la revendication 2,
- un échantillon témoin positif, tel qu'il est défini à la revendication 3, et
- un témoin négatif.

8. Kit de détection, selon la revendication 7, **caractérisé en ce que** ladite sonde fluorogène est marquée à son extrémité 5' avec un fluorochrome émetteur et à son extrémité 3' avec un fluorochrome suppresseur et un groupe MGB.

9. Paire d'amorces capable d'amplifier un quelconque génome de VHD, ladite paire d'amorces étant sélectionnée dans le groupe consistant en la paire d'amorces SEQ ID n°:1 et SEQ ID n°:2 et la paire d'amorces SEQ ID n°:3 et SEQ ID n°:2.

10. Sonde spécifique du fragment d'acide nucléique amplifié par l'une des paires d'amorces, telles qu'elles sont définies à la revendication 9, ladite sonde consistant en la séquence définie par la SEQ ID n°:4.


### SEQUENCE LISTING

<110> Assistance publique-Hopitaux de Paris
<120> Quantitative detection of HDV
<130> F1020 CAS 24 EP
<160> 4
<170> PatentIn version 3.1
<210> 1
   <211> 17
   <212> DNA
   <213> Artificial
<400> 1
   gcatggtccc agcctcc 17
<210> 2
   <211> 17
   <212> DNA
   <213> Artificial
<400> 2
   tcttcgggtc ggcatgg 17
<210> 3
   <211> 17
   <212> DNA
   <213> Artificial
<400> 3
   gcatggcccc agcctcc 17
<210> 4
   <211> 15
   <212> DNA
   <213> Artificial
<400> 4
   atgcccaggt cggac 15